# EUROPEAN PATENT APPLICATION

(11) **EP 4 722 226 A1**
(43) Date of publication of application: **08.04.2026**
(21) Application number: 25206882.0
(22) Date of filing: 06.10.2025
(51) Int. Cl.: C07K 1/14, C07K 1/34, C12N 9/00

(54) **ENZYME PURIFICATION WITH CHEMICALLY ACTIVATED CARBON**

(30) Priority: 07.10.2024 EP 24205049
(71) Applicant: BASF SE, 67056 Ludwigshafen am Rhein (DE)
(72) Inventor: Schoof, Sebastian, 67056 Ludwigshafen am Rhein (DE); Kaeding, Thomas, 67056 Ludwigshafen am Rhein (DE); Iffland, Gabriele, 67056 Ludwigshafen am Rhein (DE)
(74) Representative: Altmann Stößel Dick Patentanwälte PartG mbB

(57) **Abstract**

The present invention relates to the field of protein purification. The invention contemplates a method for purifying a fermentation-derived solution of a protein of interest comprising the following steps: contacting the fermentation-derived solution with chemically activated carbon in an amount ranging from 0.1 to 15 wt.-% of the solution; adjusting the pH to a value between 4 and 12, adjusting the temperature to a value between 2 °C to 60 °C; separating the chemically activated carbon from the solution; and thereby obtaining a clarified solution of the protein of interest. Moreover, it relates to a clarified solution of a protein of interest obtainable by or obtained by the method according to the invention and to the use of a chemically activated carbon for purifying a fermentation-derived solution of a protein of interest.

## Description

The present invention relates to the field of purification of fermentation-derived products. The invention contemplates a method for purifying a fermentation-derived solution of a protein of interest comprising the following steps: contacting the fermentation-derived solution with chemically activated carbon in an amount ranging from 0.1 to 15 wt.-% of the solution; adjusting the pH to a value between 4 and 12, adjusting the temperature to a value between 2 °C to 60 °C; separating the chemically activated carbon from the solution; and thereby obtaining a clarified solution of the protein of interest. Moreover, it relates to a clarified solution of a protein of interest obtainable by or obtained by the method according to the invention and to the use of a chemically activated carbon for purifying a fermentation-derived solution of a protein of interest.

A common way of producing a protein of interest, particularly for industrial applications, is by fermentation in an appropriate host cell followed by purification from the fermentation media. The concentrated fermentation-derived protein solutions are usually obtained by means of several sequential process steps. Purified fermentation-derived protein solutions may be regarded as a purified raw material which is either used in liquid form or may occasionally be converted into a dry form and then put to appropriate uses.

Proteins of interest, in particular enzymes, in liquid form are increasingly used for liquid and gel-like detergents and cleaning compositions. Moreover, liquid forms of proteins of interest are highly demanded in the field of cosmetics, animal nutrition, the manufacture and processing of textiles and food production for converting raw materials to end products. High quality and highly pure protein fermentation products are desired for these applications. In particular, colorization is to be avoided in the protein fermentation product in order to avoid discoloration in the end product.

Purification processes for obtaining purified fermentation-derived protein products are described in detail in the prior art. The processes typically include the removal of the biomass, i.e., the constituents of the host organisms, more particularly macromolecular constituents, the removal of low molecular weight accompanying substances and impurities, including media constituents and metabolites, and the removal of other proteins, such as enzymes. Despite the numerous process steps, it is an aim, to obtain the protein of interest in large quantities, in high purity and high quality.

DE3915277 A1 describes a process for cleaning and deodorizing aqueous enzyme solutions obtained by the fermentation of bacteria or fungi by the in situ production of a water-insoluble deposit. The adsorption of undesired admixtures to this deposit and subsequent solid/liquid separation is intended to improve the light color of the enzyme solution and the quantity of enzymes. This is achieved by adding to the enzyme solution, set and maintained at a pH of between 5 and 11, an acid aqueous solution of an aluminum salt and, if desired, other precipitating agents and a separating off water-insoluble components, whereby a masking agent selected from the group: boric acids and/or their water-soluble salts; sulphurous acids and/or their water-soluble salts; hydroxycarboxylic acids and/or their water-soluble salts; multivalent alcohols may be added.

WO2004067557 A1 discloses a process comprising the steps of: (A) providing a concentrated enzyme solution comprised of insoluble solids; (B) separating the insoluble solids to produce a solids-free supernatant solution and (C) contacting the supernatant solution with a strongly basic anion exchanger carried out with a bed volume of from 1 to 10 to produce a decolorized protein. However, depending on the isoelectric point of the enzyme, the enzyme may have a negative charge and bind to the anion exchanger, hence the method appears to be limited to decolorizing of alkaline proteins.

Hence, there is still a need for improved purification processes that result in high levels of any purified protein of interest in the desired quality. In particular, fermentation-derived solutions designed for the detergent market need to be almost colorless to full fill customer requirements.

The technical problem underlying the present invention may be regarded as the provision of means and methods for complying with the aforementioned needs. The technical problem is solved by the embodiments characterized in the claims and herein below.

Thus, the present invention relates to a method for purifying a fermentation-derived solution of a protein of interest comprising the following steps:
a) contacting the fermentation-derived solution with chemically activated carbon in an amount ranging from 0.1 to 15 wt.-% of the solution;
b) adjusting the pH to a value between 4 and 12,
c) adjusting the temperature to a value between 2 °C to 60 °C;
d) separating the chemically activated carbon from the solution; and
e) thereby obtaining a clarified solution of the protein of interest.

It is to be understood that as used in the specification and in the claims, "a" or "an" can mean one or more, depending upon the context in which it is used. Thus, for example, reference to "a host cell" can mean that at least one host cell can be utilized.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. The term "comprising" also encompasses embodiments where only the items referred to are present, i.e. it has a limiting meaning in the sense of "consisting of", or not.

Further, as used in the following, the terms "particularly", "more particularly", "typically", and "more typically" or similar terms are used in conjunction with additional or alternative features, without restricting alternative possibilities. Thus, features introduced by these terms are additional or alternative features and are not intended to restrict the scope of the claims in any way. The invention may, as the skilled person will recognize, be performed by using alternative features. Similarly, features introduced by "in an embodiment of the invention" or similar expressions are intended to be additional or alternative features, without any restriction regarding alternative embodiments of the invention, without any restrictions regarding the scope of the invention and without any restriction regarding the possibility of combining the features introduced in such way with other additional or alternative or non-additional or alternative features of the invention. Not mandatory, but preferred features are also characterized by the term "preferably", "more preferably" or "most preferably".

Further, it will be understood that the term "at least" means that the item or parameter to which the term refers is limited in one direction but open ended in one or more other directions.

Further, it will be understood that the term "at least one" as used herein means that one or more of the items referred to following the term may be used in accordance with the invention. For example, if the term indicates that at least one feed solution shall be used this may be understood as one feed solution or more than one feed solutions, i.e. two, three, four, five or any other number of feed solutions. Depending on the item the term refers to the skilled person understands as to what upper limit the term may refer, if any.

The term "about" as used herein means that with respect to any number recited after said term an interval accuracy exists within in which a technical effect can be achieved. Accordingly, about as referred to herein, preferably, refers to the precise numerical value or a range around said precise numerical value of ±20 %, preferably ±15 %, more preferably ±10 %, or even more preferably ±5 %.

Furthermore, the terms "first", "second", "third" or "(a)", "(b)", "(c)", "(d)" etc. and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. In case the terms "first", "second", "third" or "(a)", "(b)", "(c)", "(d)", "i", "ii" etc. relate to steps of a method or use or assay, there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

It is to be understood that this invention is not limited to the particular methodology, protocols, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

In the present invention, the inventors have found means and methods for purifying a fermentation-derived solution of protein of interest. In particular, the combining the steps of contacting the fermentation derived solution of a protein of interest with chemically activated carbon as described elsewhere herein with a defined adjustment of pH and temperature leads to an efficient purification method with an advantageous purification results. In particular and advantageously, undesired discoloration of the protein solution appears to be removed by the method according to the invention resulting in a particularly pure solution that fulfills the high quality standards for various downstream applications, including manufacture of detergents and cosmetics. Furthermore, the methods and means according to the invention is advantageous as ways for purifying any kind of protein of interest are provided, such as alkaline and non-alkaline proteins, independent of their isoelectric points. Still further, the purification according to the invention may contribute to enhancing stability of the purified product and formulations thereof.

The terms "protein" or "polypeptide" are used interchangeably and refer to molecules consisting of a chain of amino acids, without reference to a specific mode of action, size, 3-dimensional structure or origin. Typically, the term "polypeptide" refers to a molecule consisting of a contiguous sequence of at least 20, amino acids that are covalently linked to each other by peptide bonds. Molecules consisting of less than 20 amino acids covalently linked by peptide bonds are usually considered to be "peptides". Preferably, the polypeptide comprises of from 50 to 1000, more preferably of from 75 to 1000, still more preferably of from 100 to 500, most preferably of from 110 to 400 amino acids.

The term "protein of interest" as referred to herein refers to any protein, peptide or fragment thereof which is intended to be produced in a fermentation process. The term "protein of interest", thus, encompasses polypeptides, peptides, fragments thereof as well as fusion proteins and the like.

According to the present invention, preferably, the protein of interest is produced by a host cell, more preferably said host cell comprising an expression construct carrying a nucleic acid sequence encoding for the protein of interest, during the fermentation process.

Preferably, the protein of interest is an enzyme. In a particular embodiment, the enzyme is classified as an oxidoreductase (EC 1), a transferase (EC 2), a hydrolase (EC 3), a lyase (EC 4), an isomerase (EC 5), or a ligase (EC 6) (EC-numbering according to Enzyme Nomenclature, Recommendations (1992) of the Nomenclature Committee of the International Union of Biochemistry and Molecular Biology including its supplements published 1993-1999). In a preferred embodiment, the protein of interest is an enzyme suitable to be used in detergents.

Most preferably, the enzyme is a hydrolase (EC 3), preferably, a glycosidase (EC 3.2) or a peptidase (EC 3.4). Especially preferred enzymes are enzymes selected from the group consisting of an amylase (in particular an alpha-amylase (EC 3.2.1.1)), a cellulase (EC 3.2.1.4), a lactase (EC 3.2.1.108), a mannanase (EC 3.2.1.25), a lipase (EC 3.1.1.3), a phytase (EC 3.1.3.8), a nuclease (EC 3.1.11 to EC 3.1.31), and a protease (EC 3.4); in particular amylase, alpha-amylase, glucoamylase, pullulanase, protease, metalloprotease, peptidase, lipase, cutinase, acyl transferase, cellulase, endoglucanase, glucosidase, cellubiohydrolase, xylanase, xyloglucantransferase, xylosidase, mannanase, phytase, phosphatase, xylose isomerase, glucoase isomerase, lactase, acetolactate decarboxylase, pectinase, pectin methylesterase, polygalacturonidase, lyase, pectate lyase, arabinase, arabinofuranosidase, galactanase, a laccase, peroxidase and an asparaginase.

Enzymes of particular interest may further be selected from: protease, oxidoreductase, transferase, hydrolase, lyase, isomerase, ligase, aminopeptidase, amylase, asparaginase, carbohydrase, carboxypeptidase, catalase, cellulase, chitinase, cutinase, cyclodextrin glycosyltransferase, deoxyribonuclease, esterase, alpha-galactosidase, betagalactosidase, glucoamylase, alpha-glucosidase, beta-glucosidase, hyaluronic acid synthase, invertase, laccase, lipase, mannosidase, mutanase, oxidase, a pectinolytic enzyme, peroxidase, phytase, polyphenoloxidase, ribonuclease, transglutaminase, dispersin, and xylanase.

More particularly, the enzyme may be selected from the group consisting of: amylase, protease, lipase, lactase, mannanase, phytase, xylanase, phosphatase, glucoamylase, nuclease, and cellulase, even more particularly selected from the group consisting of: amylase, protease, lipase, mannanase, phytase, xylanase, phosphatase, glucoamylase, nuclease, and cellulase, more preferably the protein of interest is selected from amylase, and protease; even more preferably the protein of interest is a protease, for example a serine protease such as subtilisin protease.

"A fermentation-derived solution of the protein of interest" in line with the present invention is understood as any solution derivable from a fermentation process comprising the protein of interest in a solubilized state in any amount. Typically, said solution is an aqueous solution. More typically, a fermentation-derived solution of the protein of interest may be obtained by a solid-liquid separation, for example filtration, crossflow filtration and/or centrifugation at the end of a fermentation process, for example after the time of harvest. The solid liquid separation may include a step of solubilization, dilution, stabilization and/or other pretreatment. Even more typically, the fermentation-derived solution of the protein of interest is a permeate, a filtrate, centrate or a supernatant, typically obtained during solid liquid separation after fermentation. Typically, the clarified solution is object to a concentration step, such as ultra filtration or evaporation or other means for concentration known in the art. In the alternative, the fermentation broth at the time of harvest may be directly subjected as the fermentation-derived solution to the method according to the invention.

The term "nucleic acid" or "nucleic acid molecule" as used herein, includes reference to a deoxyribonucleotide (DNA) or ribonucleotide (RNA) polymer, i.e. a polynucleotide, in either single- or double-stranded form, and unless otherwise limited, encompasses known analogues having the essential nature of natural nucleotides in that they hybridize to single-stranded nucleic acids in a manner similar to naturally occurring nucleotides (e.g., peptide nucleic acids). A polynucleotide can be full-length or a sub-sequence of a native or heterologous structural or regulatory gene. Unless otherwise indicated, the term includes reference to the specified sequence as well as the complementary sequence thereof. Thus, DNAs or RNAs with backbones modified for stability or for other reasons are "polynucleotides" as that term is intended herein. Moreover, DNAs or RNAs comprising unusual bases, such as inosine, or modified bases, such as tritylated bases, to name just two examples, are "polynucleotides" as the term is used herein. It will be appreciated that a great variety of modifications have been made to DNA and RNA that serve many useful purposes known to those of skill in the art. The term "polynucleotide" as it is employed herein embraces such chemically, enzymatically or metabolically modified forms of polynucleotides, as well as the chemical forms of DNA and RNA characteristic of viruses and cells, including among other things, simple and complex cells. Every nucleic acid sequence herein that encodes a polypeptide or protein such as the protein of interest as defined herein also, by reference to the genetic code, describes every possible silent variation of the nucleic acid. The term "conservatively modified variants" applies to both amino acid and nucleic acid sequences. With respect to particular nucleic acid sequences, the term "conservatively modified variants" refers to those nucleic acids which encode identical or conservatively modified variants of the amino acid sequences due to the degeneracy of the genetic code. The term "degeneracy of the genetic code" refers to the fact that a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations" and represent one species of conservatively modified variation.

The term "vector", also referred to as "expression construct" herein, preferably, encompasses phage, plasmid, cosmids, viral vectors as well as artificial chromosomes, such as bacterial or yeast artificial chromosomes (YAC). The vector encompassing the polynucleotide of the present invention, preferably, further comprises selectable markers for propagation and/or selection in a host. The vector may be incorporated into a host cell by various techniques well known in the art. **If** introduced into a host cell, the vector may reside in the cytoplasm or may be incorporated into the genome. In the latter case, it is to be understood that the vector may further comprise nucleic acid sequences which allow for homologous recombination or heterologous insertion. Vectors can be introduced into prokaryotic or eukaryotic cells via conventional transformation or transfection techniques. The terms "transformation" and "transfection", conjugation and transduction, as used in the present context, are intended to comprise a multiplicity of prior-art processes for introducing foreign nucleic acid (for example DNA) into a host cell, including calcium phosphate, rubidium chloride or calcium chloride co-precipitation, DEAE-dextran-mediated transfection, lipofection, f-mating, natural competence, carbon-based clusters, chemically mediated transfer, electroporation or particle bombardment. Suitable methods for the transformation or transfection of host cells, including plant cells, can be found in Sambrook et al. (loc. cit.) and other laboratory manuals, such as Methods in Molecular Biology, 1995, Vol. 44, Agrobacterium protocols, Ed.: Gartland and Davey, Humana Press, Totowa, New Jersey. Alternatively, a plasmid vector may be introduced by heat shock or electroporation techniques. Should the vector be a virus, it may be packaged in vitro using an appropriate packaging cell line prior to application to host cells.

Preferably, the protein of interest is at least partially secreted by the bacterial cells into the fermentation broth, or is expressed intracellularly by the bacterial cells and only unintentionally released from the host cells into the fermentation medium, for example by lysis of the host cells.

A "host cell" as used herein is a cell that supports the production of the protein of interest, typically in a fermentation process. More typically, the host cell is recombinant, e.g. it preferably contains a heterologous polynucleotide also referred to as "expression construct" which is introduced by man by gene technology and with regard to a polynucleotide includes all those constructions brought about by man by gene technology / recombinant DNA techniques known in the art. In other words; according to the invention a host cell refer to a cell which serves as a host for an expression construct or vector for a gene encoding a protein of interest. Said expression construct may be a naturally occurring expression construct, a recombinantly introduced expression construct or a naturally occurring expression construct which has been genetically modified in the bacterial cell.

Typically, said host cell is selected from the group consisting of: a bacterial cell, a yeast cell, a fungal cell, an algal cell or a cyanobacterial cell, a non-human animal cell or a non-human mammalian cell, and a plant cell. More typically, the host cell can be selected from any one of the following organisms:

### Bacteria:

The bacterial host cell can, for example, be selected from the group consisting of the genera Escherichia, Klebsiella, Helicobacter, Bacillus, Lactobacillus, Streptococcus, Amycolatopsis, Rhodobacter, Pseudomonas, Paracoccus, Lactococcus, Ensifer or Pantoea.
gram positive: Bacillus, Streptomyces: Useful gram positive bacterial host cells include, but are not limited to, a Bacillus cell, e.g., Bacillus alkalophius, Bacillus amyloliquefaciens, Bacillus brevis, Bacillus circulans, Bacillus clausii, Bacillus coagulans, Bacillus firmus, Bacillus Jautus, Bacillus lentus, Bacillus licheniformis, Bacillus megaterium, Bacillus pumilus, Bacillus stearothermophilus, Bacillus subtilis, and Bacillus thuringiensis. Most preferred, the prokaryote is a Bacillus cell, preferably, a Bacillus cell of Bacillus subtilis, Bacillus pumilus, Bacillus licheniformis, or Bacillus lentus.

Some other preferred bacteria include strains of the order Actinomycetales, preferably, Streptomyces, preferably Streptomyces spheroides (ATTC 23965), Streptomyces thermoviolaceus (IFO 12382), Streptomyces lividans or Streptomyces murinus or Streptoverticillum verticillium ssp. verticillium. Other preferred bacteria include Rhodobacter sphaeroides, Rhodomonas palustri, Streptococcus lactis. Further preferred bacteria include strains belonging to Myxococcus, e.g., M. virescens.
gram negative: Escherichia, Pseudomonas, Rhodobacter, Paracoccus, Ensifer or Pantoea species: Preferred gram negative bacteria are Escherichia coli, Pseudomonas sp., preferably, Pseudomonas purrocinia (ATCC 15958) or Pseudomonas fluorescens (NRRL B-11) or Pseudomonas denitrificans, Rhodobacter capsulatus or Rhodobacter sphaeroides, Paracoccus carotinifaciens, Paracoccus zeaxanthinifaciens, Pantoea ananatis, or Sinorhizobium meliloti also known as Ensifer meliloti.

### Fungi:

Aspergillus, Fusarium, Trichoderma: The host cell may be a fungal cell. "Fungi" as used herein includes the phyla Ascomycota, Basidiomycota, Chytridiomycota, and Zygomycota as well as the Oomycota and Deuteromycotina and all mitosporic fungi. Representative groups of Ascomycota include, e.g., Neurospora, Eupenicillium (=Penicillium), Emericella (=Aspergillus), Eurotium (=Aspergillus), Thermothelomyces and the true yeasts listed below. Examples of Basidiomycota include mushrooms, rusts, and smuts. Representative groups of Chytridiomycota include, e.g., Allomyces, Blastocladiella, Coelomomyces, and aquatic fungi. Representative groups of Oomycota include, e.g. Saprolegniomycetous aquatic fungi (water molds) such as Achlya. Examples of mitosporic fungi include Aspergillus, Penicillium, Candida, and Alternaria. Representative groups of Zygomycota include, e.g., Rhizopus and Mucor.

Some preferred fungi include strains belonging to the subdivision Deuteromycotina, class Hyphomycetes, e.g., Fusarium, Humicola, Tricoderma, Myrothecium, Verticillum, Arthromyces, Caldariomyces, Ulocladium, Embellisia, Cladosporium or Dreschlera, in particular Fusarium oxysporum (DSM 2672), Humicola insolens, Trichoderma resii, Myrothecium verrucana (IFO 6113), Verticillum alboatrum, Verticillum dahlie, Arthromyces ramosus (FERM P-7754), Caldariomyces fumago, Ulocladium chartarum, Embellisia alli Dreschlera halodes or Thermothelomyces thermophiles (ATCC 42464) also referred to as Myceliophthora thermophila. Other preferred fungi include strains belonging to the subdivision Basidiomycotina, class Basidiomycetes, e.g. Coprinus, Phanerochaete, Coriolus or Trametes, in particular Coprinus cinereus f. microsporus (IFO 8371), Coprinus macrorhizus, Phanerochaete chrysosporium (e.g. NA-12) or Trametes (previously called Polyporus), e.g. T. versicolor (e.g. PR4 28-A).

Further preferred fungi include strains belonging to the subdivision Zygomycotina, class Mycoraceae, e.g. Rhizopus or Mucor, in particular Mucor hiemalis.

Yeast, Pichia, Saccharomyces: The fungal host cell may be a yeast cell. Yeast as used herein includes ascosporogenous yeast (Endomycetales), basidiosporogenous yeast, and yeast belonging to the Fungi Imperfecti (Blastomycetes). The ascosporogenous yeasts are divided into the families Spermophthoraceae and Saccharomycetaceae. The latter is comprised of four subfamilies, Schizosaccharomycoideae (e.g., genus Schizosaccharomyces), Nadsonioideae, Lipomycoideae, and Saccharomycoideae (e.g. genera Kluyveromyces, Pichia, and Saccharomyces). The basidiosporogenous yeasts include the genera Leucosporidim, Rhodosporidium, Sporidiobolus, Filobasidium, and Filobasidiella. Yeasts belonging to the Fungi Imperfecti are divided into two families, Sporobolomycetaceae (e.g., genera Sporobolomyces and Bullera) and Cryptococcaceae (e.g. genus Candida).

### Eukaryotes:

Eukaryotic host cells further include, without limitation, a non-human animal cell, a non-human mammal cell, an avian cell, reptilian cell, insect cell or a plant cell.

Still more preferably, said host cell is a microbial cell, such as a fungal cell, an archaea cell, an algae cell, a protozoa cell or a bacterial cell; more preferably a fungal or a bacterial cell. Bacterial host cells are preferred.

Bacterial host cells may in principle be selected from the group of bacteria consisting of Bacillus, Streptomyces, Escherichia, Buttiauxella and Pseudomonas. In the method of the invention, the host cell is preferably a cell of a Bacillus species.

According to the method of the invention, preferably, the fermentation is a bacterial fermentation, more preferably the method comprises producing the protein of interest in a Bacillus host cell.

As mentioned herein, the host cell in the method according to the invention may be a Bacillus cell. The Bacillus cell may be a cell from any member of the bacterial genus Bacillus, preferably a host cell of Bacillus licheniformis, Bacillus subtilis, Bacillus alkalophilus, Bacillus amyloliquefaciens, Bacillus brevis, Bacillus circulans, Bacillus clausii, Bacillus coagulans, Bacillus firmus, Bacillus jautus, Bacillus lentus, Bacillus megaterium, Bacillus pumilus, Bacillus stearothermophilus, Bacillus thuringiensis or Bacillus velezensis. More preferably, the Bacillus host cell is a Bacillus licheniformis, Bacillus pumilus, or Bacillus subtilis cell, even more preferred Bacillus licheniformis or Bacillus subtilis cell, most preferably, Bacillus licheniformis host cell. Particular preferably, the Bacillus licheniformis is selected from the group consisting of Bacillus licheniformis as deposited under American Type Culture Collection number ATCC 14580, ATCC 31972, ATCC 53926, ATCC 53757, ATCC 55768, and under DSMZ number (German Collection of Microorganisms and Cell Cultures GmbH) DSM 13, DSM 394, DSM 641, DSM 1913, DSM 11259, and DSM 26543.

Typically, the host cell belongs to the species Bacillus licheniformis, such as a host cell of the Bacillus licheniformis strain as deposited under American Type Culture Collection number ATCC 14580 (which is the same as DSM 13, see Veith et al. "The complete genome sequence of Bacillus licheniformis DSM 13, an organism with great industrial potential." J. Mol. Microbiol. Biotechnol. (2004) 7:204-211). Alternatively, the host cell may be a host cell of Bacillus licheniformis strain ATCC 53926. Alternatively, the host cell may be a host cell of Bacillus licheniformis strain ATCC 31972. Alternatively, the host cell may be a host cell of Bacillus licheniformis strain ATCC 53757. Alternatively, the host cell may be a host cell of Bacillus licheniformis strain ATCC 53926. Alternatively, the host cell may be a host cell of Bacillus licheniformis strain ATCC 55768. Alternatively, the host cell may be a host cell of Bacillus licheniformis strain DSM 394. Alternatively, the host cell may be a host cell of Bacillus licheniformis strain DSM 641. Alternatively, the host cell may be a host cell of Bacillus licheniformis strain DSM 1913. Alternatively, the host cell may be a host cell of Bacillus licheniformis strain DSM 11259. Alternatively, the host cell may be a host cell of Bacillus licheniformis strain DSM 26543.

A "fermentation process" comprises the cultivation of host cells, in particular the bacterial cells, more particularly the Bacillus cells, in a suitable fermentation medium, also referred to as "cultivation medium". "Cultivation of the cells" or "growth of the cells" is not understood to be limited to an exponential growth phase, but can also include the physiological state of the cells at the beginning of growth after inoculation and during a stationary phase. Typically the cultivation may take place at a cultivation temperature suitable for supporting growth of the cells. More typically, the cultivation temperature may lay within the range of 25 °C to 45 °C. Further details are specified elsewhere herein. The fermentation process may usually take place in a fermenter, preferably in a fermenter with a volume scale which is at least 1 m³.

An industrially relevant fermentation process encompasses a fermentation process on a volume scale which is 1-500 m³ with regard to the nominal fermenter size, preferably 5-500 m³, more preferably 10-500 m³, even more preferably 25-500 m³, most preferably 50-500 m³. In other words, an industrially relevant fermentation process encompasses a fermentation process on a volume scale which is at least 1000 L with regard to the nominal fermenter size, preferably at least 5,000 L, more preferably at least 10,000 L, even more preferably at least 25,000 L, still even more preferably at least 50,000 L and most preferably 50,000-500,000 L.

Typically, the fermentation process ends or is stopped when a desired titer of the protein of interest is reached. The term "titer of a protein of interest" as used herein is understood as the amount of molecule of interest in g per volume of fermentation broth in liter or in g per kg fermentation broth. The titer of a protein of interest may reach 2 to 100 g molecule / kg fermentation broth at the end of the fermentation process, e.g. at the time of harvest. Preferably, at the time of harvest the titer of a protein of interest reaches the desired range. The desired range may refer to up to 100 g product / kg fermentation broth. More preferably, at the time of harvest the titer of a protein of interest reaches 2 to 100 g product / kg fermentation broth, more preferably the protein of interest reaches 5 to 50 g product / kg fermentation broth at the time of harvest. The term "time of harvest" may particularly refer to the end of the fermentation, more particularly to the point in time when a desirable titer of the protein of interest has been reached, such as 2 to 100 g protein of interest / kg fermentation broth. Even more particularly, the time of harvest refers to the point in time prior to step (a) of the method according to the invention or the point in time prior to storage.

The terms"purifying" or "recovering" may be used interchangeably and are intended to mean "rendering more pure". They refer to a process, in particular the method according to the invention, in which the protein of interest is separated from other compounds or cells present in the fermentation-derived solution.

"Impurities" in the fermentation-derived solution typically include unconverted sugars, un-converterted substrate impurities, for example poly- and oligo-saccharides, fatty acids, Maillard products, residual salts and undesired side-products. "Impurities" may include colored compounds that result in unwanted coloration of the protein of interest.

The term "chemically activated carbon" as used herein refers to all types of carbon that is activated by chemical means. Chemically activated carbon is typically in solid form, more typically it is powdered, granular or in the form of pellets. A preferred form for use in the method according to the invention is chemically activated carbon powder.

A suitable chemical for activating carbon may be selected from an acid for example phosphoric acid, a strong base such as potassium hydroxide or sodium hydroxide, and a salt for example potassium carbonate, calcium chloride, and zinc chloride. A preferred chemical for producing an activated carbon suitable for use in the present invention is phosphoric acid. In an activation process as known in the art, the carbon is typically impregnated with the chemical and then exposed to high temperatures, such as 250-600 °C. Chemically activated carbon is commercially available for example from Norit NV (the Netherlands), Ceca S.A. (France) or Calgon Carbon Corporation (USA). A particularly suitable chemically activated carbon according to the invention may be produced from coniferous wood by chemical activation, in more particularly by chemical activation using phosphoric acid. Still more particularly, a suitable chemically activated carbon according to the invention may be produced from wood of the Pinaceae family, even more particularly from marine pine wood, for example wood of Pseudotsuga menziesii (douglas fir).

In line with the present invention, the step of separating the chemically activated carbon from the solution may comprise a solid-liquid separation such as centrifugation, filtration, crossflow filtration or sedimentation.

The term "solid-liquid separation" refers to any means and methods for separating solids from liquids in a mixture, such as a fermentation broth, that are known to the skilled artisan. A solid-liquid separation in accordance with present invention is performed for removing the bacterial cells producing the protein of interest from the fermentation broth. It may further be applied for removing other types of solids from a solution such as removing the activated carbon from the dispersion described elsewhere herein. The solid-liquid separation may typically be achieved by processes known in the art, such as centrifugation, filtration, decanting and/or sedimentation or a combination thereof.

In line with the present invention, the protein of interest may be heterologously expressed, preferably the protein of interest may be an enzyme, more preferably wherein the protein of interest is selected from the group consisting of: protease, oxidoreductase, transferase, hydrolase, lyase, isomerase, ligase, aminopeptidase, amylase, asparaginase, carbohydrase, carboxypeptidase, catalase, cellulase, chitinase, cutinase, cyclodextrin glycosyltransferase, deoxyribonuclease, esterase, alpha-galactosidase, betagalactosidase, glucoamylase, alpha-glucosidase, beta-glucosidase, hyaluronic acid synthase, invertase, laccase, lipase, mannosidase, mutanase, oxidase, a pectinolytic enzyme, peroxidase, phytase, polyphenoloxidase, ribonuclease, transglutaminase, dispersin, xylanase.

Still more preferably, the protein of interest is selected from the group consisting of: amylase, protease, lipase, mannanase, phytase, xylanase, phosphatase, glucoamylase, nuclease, and cellulase, even more preferably selected from amylase, and protease; still even more preferably the protein of interest is a protease, such as an alkaline protease. For example a protease comprising or consisting of an amino acid sequence corresponding to the amino acid sequence of SEQ ID NO:1.

The term "recombinant" is known to the skilled artisan. In particular, "recombinant" (or transgenic) with regard to a cell or an organism means that the cell or organism contains a heterologous polynucleotide also referred to as "expression construct" which is introduced by man by gene technology and with regard to a polynucleotide includes all those constructions brought about by man by gene technology / recombinant DNA techniques known in the art.

The term "heterologous" (or exogenous or foreign or recombinant or non-native) polypeptide or protein is defined herein as a polypeptide that is not native to the host cell, a polypeptide or protein native to the host cell in which structural modifications, e.g., deletions, substitutions, and/or insertions, have been made by recombinant DNA techniques to alter the native polypeptide or protein, or a polypeptide/protein native to the host cell whose expression is quantitatively altered or whose expression is directed from a genomic location different from the native host cell as a result of manipulation of the DNA of the host cell by recombinant DNA techniques, or whose expression is quantitatively altered as a result of manipulation of the regulatory elements of the polynucleotide by recombinant DNA techniques e.g., a stronger promoter; or a polynucleotide native to the host cell, but integrated not within its natural genetic environment as a result of genetic manipulation by recombinant DNA techniques. The term "heterologously expressed" hence may typically refer to a protein of interest being expressed in a host cell from a heterologous expression construct. More typically, the host cell comprises the heterologous expression construct as a result of genetic manipulation by recombinant DNA techniques. These techniques are known in the art.

With respect to two or more polynucleotide sequences or two or more amino acid sequences, the term "heterologous" is used to characterize that the two or more polynucleotide sequences or two or more amino acid sequences are naturally not occurring in the specific combination with each other.

The step of contacting the fermentation-derived solution with chemically activated carbon as used herein refers to bringing fermentation-derived solution in contact with a suitable amount of chemically activated carbon. This can be achieved, preferably, by adding the chemically activated carbon, particularly as a powder, to the solution, or by allowing the fermentation derived solution to pass through a carbon loaded filter bed, e.g. it may filtration over an activated carbon filter layer or over an activated carbon packed filter column.

In other words, said step of contacting the fermentation-derived solution with chemically activated carbon may comprise adding a suitable amount of chemically activated carbon to the fermentation-derived solution; and/or may comprise filtration over an activated carbon filter layer or over an activated carbon packed filter column. Suitable carbon packed filter systems are commercially available for example from Pall Corporation (USA).

In particular, the contacting is performed for a time and under conditions sufficient to allow for clarifying the protein of interest or the solution thereof; more particularly for a time sufficient to allow adsorption of impurities, including undesired side products such as colored compounds, to the chemically activated carbon. More particularly, the contacting is performed for a period of time (contacting time) that depends on the amount and type of protein of interest present in the solution, the amount of impurities in the solution, the host cell organism, the temperature specifically as defined elsewhere herein. Still more particularly, the contacting is performed for at least 15 seconds up to 36 h, more preferably for at least 10 minutes up to 10 hours. Said contacting typically results in decolorizing the protein of interest or the solution thereof.

The step of contacting the fermentation-derived solution with chemically activated carbon may comprise mixing, stirring or agitating the resulting dispersion. In particular in case of adding the chemically activated carbon directly to the solution, said adding may be followed by agitating, mixing or stirring the resulting dispersion of activated carbon in the fermentation derived solution, which may typically extend the contacting time.

In case of filtration over a carbon packed filter bed, the contacting time may typically range from a few seconds, such as five seconds, to several minutes depending on the volume of the fermentation-derived solution to be passed over the filter.

Typically, the amount of chemically activated carbon contacted with the fermentation-derived solution of the protein of interest ranges from 0.05 to 10 wt.-% of the solution, more typically from 0.1 to 7 wt.-% of the solution, even more typically from 0.2 to 5 wt.-% of the solution. Still more typically, the amount of chemically activated carbon contacted with the fermentation-derived solution of the protein of interest ranges from 0.2 to 2 wt.-% of the solution. Said range of the amount of chemically activated carbon is advantageous as it may contribute to superior purity of the protein of interest while avoiding undesired loss of protein of interest by absorbance to excess activated carbon.

The chemically activated carbon may be added in solid form to the fermentation derived solution, for example in form of a powder or of a particulate. Alternatively, the activated carbon may be dispersed in a suitable medium prior to adding to the fermentation-derived solution.

Typically, in the clarified solution of the protein of interest the protein of interest at a concentration in the range of 1 to 250 g/kg has a CIELAB value for L of above 50, preferably of above 60, of above 70, of above 80, of above 85; more preferably, a CIELAB value for L of 90 or even above. CIELAB values can be determined by any means known in the art. Typically, a spectrophotometer is used such as a Hach Lange DR 6000 or other commercially available spectrophotometer; more typically the CIELAB value is determined by measuring a sample of the clarified solution in a photometric cuvette such as a 1 mL polysterene cuvette.

The CIELAB scale refer to a uniform color scale that is commonly used today (CIE International Commission on Illumination, Recommendations on Uniform Color Spaces, Color-Difference Equations, Psychometric Color Terms, Supplement No. 2 to CIE Publication No. 15, Colorimetry, 1971 and 1978).

The method according to the invention may further comprise adding a stabilizing agent. Preferably, the stabilizing agent is a polyol, for example a polyvinyl alcohol or a soluble calcium compound, such as calcium chloride or calcium hydroxide.

In the method according to the invention, moreover, the adjustment of the temperature typically plays a crucial role for the purification result. The temperature may be adjusted during the entire purification process or, in particular, it may be adjusted at the time of contacting the fermentation-derived solution with chemically activated carbon, i.e. parallel with step a) and/or step b). The use of a chemically activated carbon contacted with the fermentation derived solution at a suitable temperature may have beneficial effect on the purification result. A suitable temperature according to the present invention has a value in the range of 2 °C to 60 °C, preferably a value in the range of 5°C to 45 °C, more preferably a value in the range of 8 °C to 40 °C, still more preferably of 10 °C to 25 °C.

In other words, in the method according to the invention, the temperature may be adjusted to a value between 5 °C and 45 °C, preferably between 8 °C and 40 °C, still more preferably 10 °C and 25 °C. Suitable measures for adjusting the temperature are known in the art and include for example heating elements and heaters that are capable of transferring heat to the fermentation derived solution. Typical elements and heaters are commercially available.

According to the method of the present invention, the pH is adjusted to a value between 4 and 12, preferably to a value between 4.5 and 11.5. The pH may typically be adjusted depending on the protein of interest. Preferably, the pH may be adjusted to a value between pH 4.5 and pH 7 in case the protein of interest is a protease; or to a value between pH 9 and pH 11.5 in case the protein of interest is an amylase. In a preferred embodiment, for example for purifying a protease, the pH is adjusted to a value between pH 5.5 and 6.5. More preferably, the pH is adjusted to a value between pH 5.8 and pH 6.2, more preferably to a value of about pH 6.

Adjusting the pH may be done by stepwise addition of an acid to lower the pH value or of a base to increase the pH value until the desired pH value is reached. As a suitable acid hydrochloric acid, acetic acid or formic acid may be used, a suitable base may be sodium hydroxide, potassium hydroxide or ammonium hydroxide. The addition may be automated or performed manually.

Specifically, in the method according to the invention, the amount of chemically activated carbon added to the fermentation-derived solution may range 3 to 5 wt.-% of the solution, the temperature may be adjusted to a value between 10 °C and 25 °C and the pH may be adjusted to a value between pH 5.5 and 6.5, more specifically the amount of chemically activated carbon added to the fermentation-derived solution may range 3.5 to 4.5 wt.-% of the solution and the pH may be adjusted to a value between pH 5.8 and 6.2. Still more specifically, the amount of chemically activated carbon added to the fermentation-derived solution may be about 4 wt.-% of the solution, the temperature may be adjusted to a value between 10 °C and 25 °C and the pH may be adjusted to pH 6. The combination of the amount of carbon with the specified pH advantageously results in a particularly effective purification discoloration of the protein of interest in the fermentation-derived solution. In other words the clarified solution advantageously exhibits a high CIELAB L value as defined elsewhere herein.

The method further comprises separating the chemically activated carbon from the solution and thereby obtaining a clarified solution of the protein of interest. Suitable separation techniques are known in the art and include all types of solid-liquid separation methods and means as defined elsewhere herein; in particular, centrifugation, filtration, crossflow filtration, decanting, sedimentation.

The method may further comprise repeating steps a) to d) at least two times. This may increase the purification, in particular the discoloration effect.

Typically, the amount of protein of interest in the clarified solution is at least 75 % of the amount of protein of interest present in the fermentation-derived solution.

The term "clarified solution" in line with the present invention refers to a solution, typically an aqueous solution comprising the protein of interest, wherein said solution and/or said protein of interest has been purified, typically by performing the method according to the invention. More typically, the clarified solution can be obtained from a fermentation-derived solution comprising the protein of interest that has been purified in line with the present invention. Hence, a clarified solution is specifically obtained in the method according to the invention. Compared to the fermentation-derived solution, the clarified solution typically contains less impurities such as colored compounds. Hence, the clarified solution more typically has a higher CIELAB L value than the fermentation derived solution. Even more typically, the clarified solution of the protein of interest at a concentration in the range of 1 to 250 g/kg has a CIELAB value for L of above 50, of above 60, of above 70, of above 80, or of above 85, of above 90, of above 95, or even higher. Still more typically, the clarified solution of the protein of interest has a concentration of the protein of interest in the range of 50 to 250 g/kg; still even more typically at a concentration in the range of 50 to 250 g/kg the clarified solution of the protein of interest has a CIELAB value for L of above 50, of above 60, of above 70, of above 80, or of above 85, of above 90, of above 95, or even higher.

Preferably, the clarified solution of the protein of interest exhibits a CIELAB value for a and/or for b that approaches 0; in other words the clarified solution of the protein of interest exhibits a CIELAB value for a and/or for b that is closer to 0 than that of a fermentation-derived solution prior to purification by the method of the invention. More preferably, the clarified solution of the protein of interest exhibits a CIELAB value for a and/or b in the range of -20 to 20, i.e. -20< a < 20 and/or -20< b < 20. Still more preferably, the clarified solution of the protein of interest exhibits a CIELAB value for a that approaches 0; in other words the clarified solution of the protein of interest exhibits a CIELAB value for a that is closer to 0 than that of a fermentation-derived solution prior to purification by the method of the invention.

The present invention further contemplates a clarified solution of a protein of interest obtainable by or obtained by the method of the present invention.

Moreover the present invention relate to the use of a chemically activated carbon for purifying a fermentation-derived solution of a protein of interest. In particular, the use comprises reducing the amount of colored compounds in the fermentation-derived solution of a protein of interest, more particularly, the use for reducing the amount of colored compounds in the fermentation-derived solution of a protein of interest.

Advantageously, it has been found in accordance with the present invention that unwanted coloration of a fermentation-derived solution of the protein of interest can be massively reduced by chemically activated carbon treatment in particular if combined with suitable temperature and pH conditions.

The explanations and interpretations of the terms given above in this specification apply for all embodiments characterized herein. The following embodiments are particular preferred embodiments according to the present invention:
1. A method for purifying a fermentation-derived solution of a protein of interest comprising the following steps:
   a) contacting the fermentation-derived solution with chemically activated carbon in an amount ranging from 0.1 to 15 wt.-% of the solution;
   b) adjusting the pH to a value between 4 and 12,
   c) adjusting the temperature to a value between 2 °C to 60 °C;
   d) separating the chemically activated carbon from the solution; and
   e) thereby obtaining a clarified solution of the protein of interest.
2. The method according to any one of the preceding embodiment, wherein the step of separating the chemically activated carbon from the solution comprises a solid-liquid separation such as centrifugation, filtration or sedimentation.
3. The method according to any one of the preceding embodiments, wherein the protein of interest is heterologously expressed, preferably wherein the protein of interest is an enzyme, more preferably wherein the protein of interest is selected from the group consisting of: protease, oxidoreductase, transferase, hydrolase, lyase, isomerase, ligase, aminopeptidase, amylase, asparaginase, carbohydrase, carboxypeptidase, catalase, cellulase, chitinase, cutinase, cyclodextrin glycosyltransferase, deoxyribonuclease, esterase, alpha-galactosidase, betagalactosidase, glucoamylase, alpha-glucosidase, beta-glucosidase, hyaluronic acid synthase, invertase, laccase, lipase, mannosidase, mutanase, oxidase, a pectinolytic enzyme, peroxidase, phytase, polyphenoloxidase, ribonuclease, transglutaminase, dispersin, xylanase, still more preferably the protein of interest is selected from the group consisting of: amylase, protease, lipase, mannanase, phytase, xylanase, phosphatase, glucoamylase, nuclease, and cellulase, more preferably selected from amylase, and protease; even more preferably the protein of interest is a protease.
4. The method according to any one of the preceding embodiments, wherein the clarified solution of the protein of interest at a concentration in the range of 1 to 250 g/kg has a CIELAB value for L of above 50.
5. The method according to any one of the preceding embodiments, wherein the method further comprises adding a stabilizing agent; preferably the stabilizing agent is a polyol or a soluble calcium compound, such as calcium chloride or calcium hydroxide.
6. The method according to any one of the preceding embodiments, wherein the amount of chemically activated carbon contacted with the fermentation-derived solution of the protein of interest ranges from 00.5 to 10 wt.-% of the solution; preferably 0.2 to 5 wt.-% of the solution.
7. The method according to any one of the preceding embodiments, wherein the temperature is adjusted to a value between 5°C and 45 °C, preferably between 8 °C and 40 °C, still more preferably 10 °C and 25 °C.
8. The method according to any one of the preceding embodiments, wherein the pH is adjusted to a value between 4.5 and 11.5, preferably to a value between pH 4.5 and pH 7 in case the protein of interest is a protease or to a value between pH 9 and pH 11.5 in case the protein of interest is an amylase.
9. The method according to any one of the preceding embodiments, wherein the step of contacting the fermentation-derived solution with chemically activated carbon comprises:
   - adding a suitable amount of chemically activated carbon to the fermentation-derived solution; and/or
   - filtration over an activated carbon filter layer or over an activated carbon packed filter column.
10. The method according to any one of the preceding embodiments, comprising repeating steps a) to d) at least two times.
11. The method according to any one of the preceding embodiments, wherein the amount of protein of interest in the clarified solution is at least 75 % of the amount of protein of interest present in the fermentation-derived solution.
12. The method according to any one of the preceding embodiments, wherein the fermentation is a bacterial fermentation, preferably comprising producing the protein of interest in a Bacillus host cell.
13. The method according to the preceding embodiment, wherein the Bacillus host cell is a cell of Bacillus licheniformis, Bacillus subtilis, Bacillus alkalophilus, Bacillus amyloliquefaciens, Bacillus brevis, Bacillus circulans, Bacillus clausii, Bacillus coagulans, Bacillus firmus, Bacillus jautus, Bacillus lentus, Bacillus megaterium, Bacillus pumilus, Bacillus stearothermophilus, Bacillus thuringiensis or Bacillus velezensis; preferably Bacillus licheniformis.
14. The method according to any one of the preceding embodiments, wherein the step of contacting the fermentation-derived solution with chemically activated carbon is performed for at least 15 seconds to 36 hours, preferably for at least 10 minutes to 10 hours.
15. The method according to any one of the preceding embodiments, wherein the step of contacting the fermentation-derived solution with chemically activated carbon comprises mixing, stirring or agitating the resulting dispersion.
16. A clarified solution of a protein of interest obtainable by or obtained by the method of any of the preceding embodiments, preferably wherein the protein of interest is an enzyme.
17. Use of an chemically activated carbon for purifying a fermentation-derived solution of a protein of interest.
18. Use according to embodiment 17, comprising reducing the amount of colored compounds in the fermentation-derived solution of a protein of interest, preferably wherein the protein of interest is an enzyme.

All references are herewith incorporated by reference in their entireties as well as with respect to the disclosure content specifically mentioned in this specification.

### FIGURES

Fig. 1: is a graphical representation of the results of example 1.
Fig. 2: is a graphical representation of the results of example 2.
Fig. 3: is a graphical representation of the results of example 3.

### EXAMPLES

The Examples shall merely illustrate the invention. They shall by no means construed as limiting the scope.

### Example 1: Decolorization of fermentation broth containing protease enzyme

Enzyme concentrate generation started with Fermentation of Bacillus licheniformis ATCC53926 comprising a gene encoding an alkaline protease from Bacillus lentus (BLAP) as described in WO9102792 comprising the mutation R99E. The amino acid sequence of said protease is specified herein as SEQ ID NO:1.

Fermentation media was prepared as described in WO2021/001297 A1.

Cultivation was conducted in a 20L stainless steel fermenter in Fed-batch culture for 36h at 37°C and pH=7.5 controlled with NH₄OH solution and feeding of Glucose maintaining an OUR of 80-100 mmol/(L*h) after consumption of initial 5 g/L of glucose.

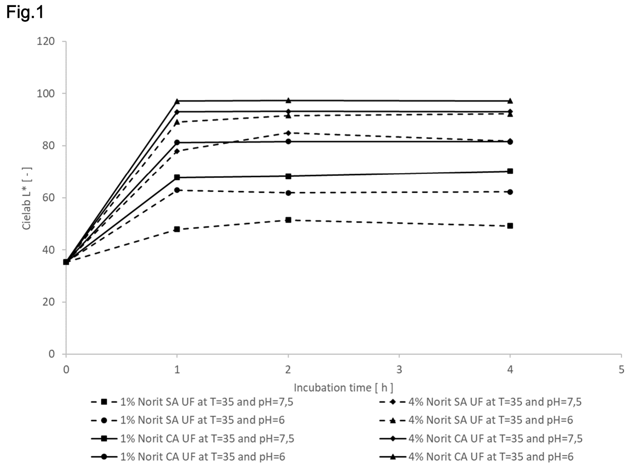

To explore decolorization with different absorbance at different conditions, 15 g of fermentation broth were incubated with amounts of activated carbon and conditions as described in figure 1. Chemically activated carbon material Norit CA UF and steam activated material Norit SA UF was purchased from Norit Nederland B.V., Amersfoort, Netherlands. pH was adjusted with 10% NaOH or acetic acid, respectively. After incubation time, the material was clarified with a 0.2 µm Chromafil RC-20/25 for CIELAB determination with a Hach Lange DR6000 Spectrophotometer.

**Table 1: Color Determination via CIELAB along decolorization of enzyme concentrate:**

| CIELAB L* after time (h) | | | | |
|---|---|---|---|---|
| | 0 | 1 | 2 | 4 |
| 1% Norit SA UF at T=35 and pH=7,5 | 35,3 | 47,9 | 51,4 | 49,2 |
| 4% Norit SA UF at T=35 and pH=7,5 | 35,3 | 78 | 84,9 | 81,8 |
| 1% Norit CA UF at T=35 and pH=7,5 | 35,3 | 67,8 | 68,2 | 70,2 |
| 4% Norit CA UF at T=35 and pH=7,5 | 35,3 | 93 | 93,2 | 93,1 |
| 1% Norit SA UF at T=35 and pH=6 | 35,3 | 62,9 | 61,8 | 62,2 |
| 4% Norit SA UF at T=35 and pH=6 | 35,3 | 89,1 | 91,6 | 92,2 |
| 1% Norit CA UF at T=35 and pH=6 | 35,3 | 81,3 | 81,6 | 81,5 |
| 4% Norit CA UF at T=35 and pH=6 | 35,3 | 97,1 | 97,4 | 97,2 |

| | CIELAB a* after time (h) | | | |
|---|---|---|---|---|
| | 0 | 1 | 2 | 4 |
| 1% Norit SA UF at T=35 and pH=7,5 | 22,5 | 15,6 | 14,8 | 15,6 |
| 4% Norit SA UF at T=35 and pH=7,5 | 22,5 | -0,6 | -1,9 | -1,5 |
| 1% Norit CA UF at T=35 and pH=7,5 | 22,5 | 5 | 3,5 | 4,3 |
| 4% Norit CA UF at T=35 and pH=7,5 | 22,5 | -2,6 | -2,8 | -3,1 |
| 1% Norit SA UF at T=35 and pH=6 | 18,3 | 9,6 | 9,4 | 9,6 |
| 4% Norit SA UF at T=35 and pH=6 | 18,3 | -1,9 | -2 | -2,1 |
| 1% Norit CA UF at T=35 and pH=6 | 18,3 | -1 | -1,3 | -1,5 |
| 4% Norit CA UF at T=35 and pH=6 | 18,3 | -2 | -2 | -2,2 |

| | CIELAB b* after time (h) | | | |
|---|---|---|---|---|
| | 0 | 1 | 2 | 4 |
| 1% Norit SA UF at T=35 and pH=7,5 | 54,4 | 59,4 | 61,4 | 61,6 |
| 4% Norit SA UF at T=35 and pH=7,5 | 54,4 | 28,3 | 23,7 | 24 |
| 1% Norit CA UF at T=35 and pH=7,5 | 54,4 | 53,7 | 52,4 | 56,4 |
| 4% Norit CA UF at T=35 and pH=7,5 | 54,4 | 13,7 | 14 | 15,4 |
| 1% Norit SA UF at T=35 and pH=6 | 68,2 | 62,4 | 61,3 | 62 |
| 4% Norit SA UF at T=35 and pH=6 | 68,2 | 17,6 | 14,8 | 13,4 |
| 1% Norit CA UF at T=35 and pH=6 | 68,2 | 46,8 | 46,7 | 47,3 |
| 4% Norit CA UF at T=35 and pH=6 | 68,2 | 7,7 | 7,5 | 7,9 |

### Example 2: Decolorization of enzyme concentrate

Enzyme concentrate generation started with Fermentation of Bacillus licheniformis ATCC53926 comprising a gene encoding the alkaline protease as described for example 1 above.

Fermentation media was prepared as described in WO2021/001297 A1.

Cultivation was conducted in a 20L stainless steel fermenter in Fed-batch culture for 36h at 37°C and pH=7.5 controlled with NH4OH solution and feeding of Glucose maintaining an OUR of 80-100 mmol/(L*h) after consumption of initial 5 g/L of glucose.

After fermentation end, the pH of fermentation broth was set to pH=7.5 with 10% NaOH solution and 8 kg broth was subject to a micro filtration dia-filtration at constant volume using a atech AlO₃ single channel 0.2 µm cut-off membrane with a channel diameter of d=6 mm and a length of 1.2m. Cross flow velocity was set to 5.5m/s, T=35°C and transmembrane pressure to TMP=1.3 bar. De-ionized water was used as dia filtration medium for a total dia-filtration factor of DF=4 related to initial broth volume. After a filtration of DF=2 was completed, pH of broth was adjusted to pH=9. Resulting clarified enzyme solution was concentrated with a concentration factor CF=30 and dia-filtered with de-ionized water with a diafiltration factor of DF=3 with a Satorius 10kDa PES membrane cassette. Clarified enzyme concentrate was stabilized with 10% mono propylene glycol.

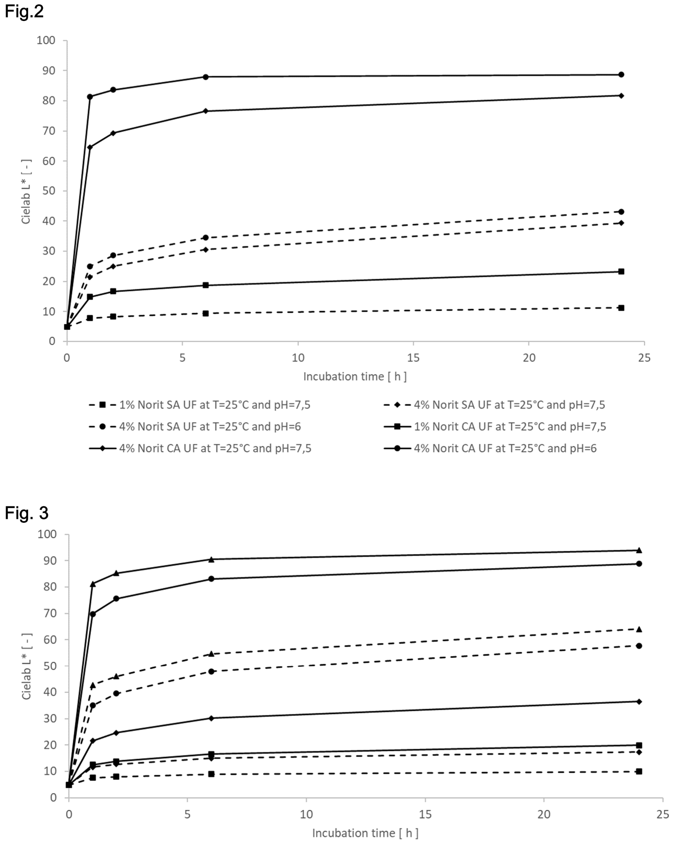

Cielab value of the material was determined with a Hach Lange DR6000 Spectrophotometer at L*=4.8; a*=20.9; b*=7.7. To explore decolorization with different absorbance at different conditions, 15g of stabilized enzyme concentrate were incubated with amounts of activated carbon and conditions as described in figure 2. Chemically activated carbon material Norit CA UF and steam activated material Norit SA UF was purchased from Norit Nederland B.V., Amersfoort, Netherlands. pH was adjusted with 10% NaOH or acetic acid, respectively. After incubation time, the material was clarified with a 0.2µm Chromafil RC-20/25 for CIELAB determination with a Hach Lange DR6000 Spectrophotometer.

**Table 2: Color Determination via CIELAB along decolorization of enzyme concentrate:**

| | CIELAB L* after time (h) | | | | |
|---|---|---|---|---|---|
| | 0 | 1 | 2 | 6 | 24 |
| 1% Norit SA UF at T=25°C and pH=7,5 | 4,8 | 7,8 | 8,3 | 9,4 | 11,3 |
| 4% Norit SA UF at T=25°C and pH=7,5 | 4,8 | 21,5 | 25 | 30,6 | 39,4 |
| 4% Norit SA UF at T=25°C and pH=6 | 4,8 | 25 | 28,6 | 34,5 | 43,1 |
| 1% Norit CA UF at T=25°C and pH=7,5 | 4,8 | 14,9 | 16,7 | 18,7 | 23,3 |
| 4% Norit CA UF at T=25°C and pH=7,5 | 4,8 | 64,6 | 69,3 | 76,6 | 81,7 |
| 4% Norit CA UF at T=25°C and pH=6 | 4,8 | 81,4 | 83,6 | 87,9 | 88,7 |

| | CIELAB a* after time (h) | | | | |
|---|---|---|---|---|---|
| | 0 | 1 | 2 | 6 | 24 |
| 1% Norit SA UF at T=25°C and pH=7,5 | 20,7 | 24,9 | 25,7 | 25,7 | 26,6 |
| 4% Norit SA UF at T=25°C and pH=7,5 | 20,7 | 26,3 | 26,1 | 25,2 | 23,1 |
| 4% Norit SA UF at T=25°C and pH=6 | 20,7 | 25,7 | 25,2 | 23,7 | 20,7 |
| 1% Norit CA UF at T=25°C and pH=7,5 | 20,7 | 26,9 | 27,1 | 27,5 | 27,8 |
| 4% Norit CA UF at T=25°C and pH=7,5 | 20,7 | 8,5 | 5,4 | 1,7 | -0,9 |
| 4% Norit CA UF at T=25°C and pH=6 | 20,7 | 0,1 | -0,8 | -2 | -2,2 |

| | CIELAB b* after time (h) | | | | |
|---|---|---|---|---|---|
| | 0 | 1 | 2 | 6 | 24 |
| 1% Norit SA UF at T=25°C and pH=7,5 | 7,6 | 12,7 | 13,7 | 15,6 | 18,7 |
| 4% Norit SA UF at T=25°C and pH=7,5 | 7,6 | 35,6 | 41 | 49,2 | 59 |
| 4% Norit SA UF at T=25°C and pH=6 | 7,6 | 41 | 46,3 | 53,8 | 60 |
| 1% Norit CA UF at T=25°C and pH=7,5 | 7,6 | 24,9 | 27,7 | 31,2 | 38,8 |
| 4% Norit CA UF at T=25°C and pH=7,5 | 7,6 | 54,2 | 50,1 | 44,5 | 38,7 |
| 4% Norit CA UF at T=25°C and pH=6 | 7,6 | 37,2 | 32,4 | 26,2 | 21,9 |

### Example 3: Decolorization of enzyme concentrate

Enzyme concentrate generation started with Fermentation of Bacillus licheniformis ATCC53926 comprising a gene encoding the alkaline protease as described for example 1 above.

Fermentation media was prepared as described in WO2021/001297 A1.

Cultivation was conducted in a 20L stainless steel fermenter in Fed-batch culture for 36h at 37°C and pH=7.5 controlled with NH4OH solution and feeding of Glucose maintaining an OUR of 80-100 mmol/(L*h) after consumption of initial 5 g/L of glucose.

After fermentation end, the pH of fermentation broth was set to pH=7.5 with 10% NaOH solution and 8kg broth was subject to a micro filtration dia-filtration at constant volume using a atech AlO₃ single channel 0.2 µm cut-off membrane with a channel diameter of d=6 mm and a length of 1.2 m. Cross flow velocity was set to 5.5 m/s, T=35°C and transmembrane pressure to TMP=1.3 bar. De-ionized water was used as dia filtration medium for a total dia-filtration factor of DF=4 related to initial broth volume. After a filtration of DF=2 was completed, pH of broth was adjusted to pH=9. Resulting clarified enzyme solution was concentrated with a concentration factor CF=30 and dia-filtered with de-ionized water with a diafiltration factor of DF=3 with a Satorius 10kDa PES membrane cassette. Clarified enzyme concentrate was stabilized with 10% mono propylene glycol. Cielab value of the material was determined with a Hach Lange DR6000 Spectrophotometer at L*=4.8; a*=20.9; b*=7.7. To explore decolorization with different absorbance at different conditions, 15g of stabilized enzyme concentrate were incubated with amounts of activated carbon and conditions as described in figure 3. Chemically activated carbon material Acticarbone HPX5 and steam activated material Acticarbone XT50 was purchased from Ceca, La Garenne-Colombes Cedex, France. pH was adjusted with 10% NaOH or acetic acid, respectively. After incubation time, the material was clarified with a 0.2 µm Chromafil RC-20/25 for CIELAB determination with a Hach Lange DR6000 Spectrophotometer.

**Table 3: Color Determination via CIELAB along decolorization of enzyme concentrate:**

| | CIELAB L* after time (h) | | | | |
|---|---|---|---|---|---|
| | 0 | 1 | 2 | 6 | 24 |
| 0,5% Ceca Acticarbone HPX5 at T=25°C and pH=7,5 | 4,8 | 12,5 | 13,8 | 16,6 | 19,9 |
| 1% Ceca Acticarbone HPX5 at T=25°C and pH=7,5 | 4,8 | 21,6 | 24,6 | 30,2 | 36,5 |
| 4% Ceca Acticarbone HPX5 at T=25°C and pH=7,5 | 4,8 | 69,8 | 75,6 | 83,1 | 88,8 |
| 4% Ceca Acticarbone HPX5 at T=25°C and pH=6 | 4,8 | 81,2 | 85,3 | 90,5 | 94 |
| 0,5% Ceca Acticarbone XT 50 at T=25°C and pH=7,5 | 4,8 | 7,6 | 8 | 9 | 9,9 |
| 1% Ceca Acticarbone XT 50 at T=25°C and pH=7,5 | 4,8 | 11,6 | 12,7 | 15 | 17,3 |
| 4% Ceca Acticarbone XT 50 at T=25°C and pH=7,5 | 4,8 | 35 | 39,5 | 47,9 | 57,8 |
| 4% Ceca Acticarbone XT 50 at T=25°C and pH=6 | 4,8 | 42,8 | 46 | 54,7 | 64,1 |

| | CIELAB a* after time (h) | | | | |
|---|---|---|---|---|---|
| | 0 | 1 | 2 | 6 | 24 |
| 0,5% Ceca Acticarbone HPX5 at T=25°C and pH=7,5 | 20,9 | 26,7 | 27 | 27,7 | 28,2 |
| 1% Ceca Acticarbone HPX5 at T=25°C and pH=7,5 | 20,9 | 27,1 | 26,9 | 26,2 | 24,9 |
| 4% Ceca Acticarbone HPX5 at T=25°C and pH=7,5 | 20,9 | 5,4 | 2,6 | -0,4 | -2,3 |
| 4% Ceca Acticarbone HPX5 at T=25°C and pH=6 | 20,9 | 0,3 | -1,1 | -2,1 | -2,4 |
| 0,5% Ceca Acticarbone XT 50 at T=25°C and pH=7,5 | 20,9 | 24,9 | 25,1 | 25,7 | 26,2 |
| 1% Ceca Acticarbone XT 50 at T=25°C and pH=7,5 | 20,9 | 26,1 | 26,3 | 27,1 | 27,6 |
| 4% Ceca Acticarbone XT 50 at T=25°C and pH=7,5 | 20,9 | 22,5 | 20,7 | 17,1 | 12,3 |
| 4% Ceca Acticarbone XT 50 at T=25°C and pH=6 | 20,9 | 18,2 | 15,5 | 10,8 | 5,7 |

| | CIELAB b* after time (h) | | | | |
|---|---|---|---|---|---|
| | 0 | 1 | 2 | 6 | 24 |
| 0,5% Ceca Acticarbone HPX5 at T=25°C and pH=7,5 | 7,7 | 20,7 | 22,9 | 27,7 | 33,3 |
| 1% Ceca Acticarbone HPX5 at T=25°C and pH=7,5 | 7,7 | 35,9 | 40,6 | 48,8 | 56,8 |
| 4% Ceca Acticarbone HPX5 at T=25°C and pH=7,5 | 7,7 | 46,9 | 42,3 | 35,5 | 29 |
| 4% Ceca Acticarbone HPX5 at T=25°C and pH=6 | 7,7 | 35,5 | 30,2 | 23 | 17 |
| 0,5% Ceca Acticarbone XT 50 at T=25°C and pH=7,5 | 7,7 | 12,5 | 13,1 | 14,9 | 16,4 |
| 1% Ceca Acticarbone XT 50 at T=25°C and pH=7,5 | 7,7 | 19,3 | 21 | 25,1 | 29 |
| 4% Ceca Acticarbone XT 50 at T=25°C and pH=7,5 | 7,7 | 53,1 | 56,2 | 58,5 | 58,9 |
| 4% Ceca Acticarbone XT 50 at T=25°C and pH=6 | 7,7 | 56 | 54,9 | 53,5 | 49,3 |

## Claims

1. A method for purifying a fermentation-derived solution of a protein of interest comprising the following steps:
a) contacting the fermentation-derived solution with chemically activated carbon in an amount ranging from 0.1 to 15 wt.-% of the solution;
b) adjusting the pH to a value between 4 and 12,
c) adjusting the temperature to a value between 2 °C to 60 °C;
d) separating the chemically activated carbon from the solution; and
e) thereby obtaining a clarified solution of the protein of interest;
wherein the protein of interest is an enzyme.

2. The method according to claim 1, wherein the step of separating the chemically activated carbon from the solution comprises a solid-liquid separation such as centrifugation, filtration or sedimentation.

3. The method according to any one of the preceding claims, wherein the protein of interest is heterologously expressed.

4. The method according to any one of the preceding claims, wherein more preferably wherein the protein of interest is selected from the group consisting of: protease, oxidoreductase, transferase, hydrolase, lyase, isomerase, ligase, aminopeptidase, amylase, asparaginase, carbohydrase, carboxypeptidase, catalase, cellulase, chitinase, cutinase, cyclodextrin glycosyltransferase, deoxyribonuclease, esterase, alpha-galactosidase, betagalactosidase, glucoamylase, alpha-glucosidase, beta-glucosidase, hyaluronic acid synthase, invertase, laccase, lipase, mannosidase, mutanase, oxidase, a pectinolytic enzyme, peroxidase, phytase, polyphenoloxidase, ribonuclease, transglutaminase, dispersin, xylanase, still more preferably the protein of interest is selected from the group consisting of: amylase, protease, lipase, mannanase, phytase, xylanase, phosphatase, glucoamylase, nuclease, and cellulase, more preferably selected from amylase, and protease; even more preferably the protein of interest is a protease.

5. The method according to any one of the preceding claims, wherein the method further comprises adding a stabilizing agent; preferably the stabilizing agent is a polyol or a soluble calcium compound, such as calcium chloride or calcium hydroxide.

6. The method according to any one of the preceding claims, wherein the amount of chemically activated carbon contacted with the fermentation-derived solution of the protein of interest ranges from 00.5 to 10 wt.-% of the solution, preferably from 0.2 to 5 wt.-% of the solution.

7. The method according to any one of the preceding claims, wherein the temperature is adjusted to a value between 5°C and 45 °C, preferably between 8 °C and 40 °C, still more preferably 10 °C and 25 °C.

8. The method according to any one of the preceding claims, wherein the pH is adjusted to a value between 4.5 and 11.5, preferably to a value between pH 4.5 and pH 7 in case the protein of interest is a protease or to a value between pH 9 and pH 11.5 in case the protein of interest is an amylase.

9. The method according to any one of the preceding claims, wherein the step of contacting the fermentation-derived solution with chemically activated carbon comprises:
- adding a suitable amount of chemically activated carbon to the fermentation-derived solution; and/or
- filtration over an activated carbon filter layer or an activated carbon packed filter column.

10. The method according to any one of the preceding claims, comprising repeating steps a) to d) at least two times.

11. The method according to any one of the preceding claims, wherein the amount of protein of interest in the clarified solution is at least 80 % of the amount of protein of interest present in the fermentation-derived solution.

12. The method according to any one of the preceding claims, wherein the fermentation is a bacterial fermentation, preferably comprising producing the protein of interest in a Bacillus host cell.

13. The method according to any one of the preceding claims, wherein the Bacillus host cell is a cell of Bacillus licheniformis, Bacillus subtilis, Bacillus alkalophilus, Bacillus amyloliquefaciens, Bacillus brevis, Bacillus circulans, Bacillus clausii, Bacillus coagulans, Bacillus firmus, Bacillus jautus, Bacillus lentus, Bacillus megaterium, Bacillus pumilus, Bacillus stearothermophilus, Bacillus thuringiensis or Bacillus velezensis; preferably Bacillus licheniformis.

14. A clarified solution of a protein of interest obtainable by or obtained by the method of any of the preceding claims; wherein the protein of interest is an enzyme.

15. Use of a chemically activated carbon for purifying a fermentation-derived solution of a protein of interest; wherein the protein of interest is an enzyme.
